(19) Europäisches Patentamt
European Patent Office
Office européen des brevets

(11) Publication number: 0 612 761 A1

(12) EUROPEAN PATENT APPLICATION

(21) Application number: 94102891.2

(22) Date of filing: 25.02.94

(51) Int. Cl.5: C07K 3/28, C12N 15/14

(30) Priority: 25.02.93 JP 37031/93
25.02.93 JP 37032/93

(43) Date of publication of application:
31.08.94 Bulletin 94/35

(84) Designated Contracting States:
BE CH DE DK ES FR GB IT LI NL SE

(71) Applicant: THE GREEN CROSS CORPORATION
3-3, Imabashi 1-chome
Chuo-ku
Osaka-shi Osaka 541 (JP)

(72) Inventor: Sumi, Akinori, c/o The Green Cross
Corp. Central
Res. Lab.,
2-25-1, Shodai-Ohtani
Hirakata-shi, Osaka (JP)
Inventor: Ishikawa, Syoichi, c/o The Green
Cross Corp.
Osadano Plant,
2-11, Osadanocho
Fukuchiyama-shi, Kyoto (JP)
Inventor: Kondo, Masahide, c/o The Green
Cross Corp.
Osadano Plant,
2-11, Osadanocho
Fukuchiyama-shi, Kyoto (JP)

Inventor: Noda, Munehiro, c/o The Green
Cross Corp. Central
Res. Lab.,
2-25-1, Shodai-Ohtani
Hirakata-shi, Osaka (JP)
Inventor: Fujiwara, Nagatoshi, c/o The Green
Cross Corp.
Miyakojima Plant,
3-5-44, Miyakojimanakadori
Miyakojima-ku, Osaka-shi, Osaka (JP)
Inventor: Ohmura, Takao, c/o The Green
Cross Corp. Central
Res. Lab.,
2-25-1, Shodai-Ohtani
Hirakata-shi, Osaka (JP)
Inventor: Yokoyama, Kazumasa, c/o The
Green Cross Corp.
Central Res. Lab.,
2-25-1, Shodai-Ohtani
Hirakata-shi, Osaka (JP)

(74) Representative: Hansen, Bernd, Dr.
Dipl.-Chem. et al
Hoffmann, Eitle & Partner,
Patentanwälte,
Arabellastrasse 4
D-81925 München (DE)

(54) Human serum albumin and process for producing the same.

(57) Human serum albumin obtained by gene manipulation techniques can be purified by treating recombinant human serum albumin with a hydrophobic chromatography carrier at pH of 2 to 5 and a salt concentration of 0.4 to 1 and exposing the carrier to a pH of 6 to 8 and a salt concentration of 0.01 to 0.3 M, or treating the culture supernatant with boric acid or a salt thereof at pH 8 to 11 for 1 to 10 hours and recovering the supernatant to thereby obtain human serum albumin which contains substantially no contaminants which are contained in the culture medium or contained in or secreted by the host microorganism, specifically free nonantigenic contaminants detectable by the phenol-sulfuric acid method, antigenic producer host-derived contaminants and pyrogen. The thus-obtained human serum albumin is of very high purity and free from various side effects attributed to the contaminants.

## FIELD OF THE INVENTION

The instant invention relates to a recombinant human serum albumin having novel properties and a process for producing said human serum albumin.

## BACKGROUND OF THE INVENTION

Albumin, especially human serum albumin (HSA), is an important protein of the circulatory system. The protein is produced in the liver and has a major role in maintaining normal osmotic pressure of body fluids, such as blood. It also serves as a carrier of various molecules.

HSA is administered under various clinical conditions, For example, in the case of shock or burn injury, HSA functions to restore blood volume and to alleviate other injury-related symptoms. Patients suffering from hypoproteinemia and fetal erythroblastosis sometimes require HSA treatment.

In other words, a common indication for HSA administration is a loss of body fluids, such as during a surgical procedure, shock, burn injury or hypoproteinemia which causes edema.

Currently, HSA is produced mainly as a fractionated product of collected blood. Such a production process, however, has disadvantages in that it is not economical and the supply of blood is sporadic. In addition, collected blood sometimes contains undesirable substances, such as hepatitis virus. In consequence, it is profitable to develop a material which can be used as an HSA substitute.

Recent advances in recombinant DNA techniques have rendered possible microbial production of various polypeptides. With regard to HSA, establishing techniques for the large scale production of HSA by recombinant methods and subsequent high grade purification also is in progress.

However, in the case of producing HSA by means of gene manipulation techniques, it is highly probable that an HSA preparation of interest will be contaminated by certain components, which are contained in the raw materials or secreted by a microorganism during culturing of the host microorganism or are introduced during purification of the resulting HSA, in the free state or with bound to other components. Removal of such contaminants is a unique problem for recombinant HSA since the contaminants are not found in plasma-derived HSA which has been conventionally used. Thus, a new method for purifying recombinant HSA has been desired to solve the above problems.

## SUMMARY OF THE INVENTION

An object of the instant invention is to provide human serum albumin obtained by means of gene manipulation techniques, from which producer host-related substances or other contaminants are removed and to provide a process for producing the same.

Taking the aforementioned problems into consideration, the instant inventors have conducted intensive studies and, as a result, succeeded in producing a recombinant HSA of high purity which does not contain free nonantigenic contaminants which is detectable by the phenol-sulfuric acid method by conducting hydrophobic chromatography under specified conditions during the purification procedure for recovering recombinant HSA. The inventors have also found that HSA of high purity from which contaminants are sufficiently removed can be obtained by treating recombinant HSA with boric acid or a salt thereof and subjecting the resulting HSA to ultrafiltration using a membrane having a specified molecular weight exclusive limit. The recombinant HSA according to the present invention is a novel substance. An HSA preparation containing the HSA which does not contain free nonantigenic contaminants detectable by the phenol-sulfuric acid method is so safe as to be free from various side effects attributed to the contaminants.

More specifically, the instant invention provides:

(1) a recombinant human serum albumin wherein a content of free nonantigenic contaminants detectable by the phenol-sulfuric acid method is 1 $\mu$g or less per 250 mg of said albumin;

(2) the albumin as mentioned above wherein a content of producer host-related antigenic contaminants is 0.1 ng or less and a content of pyrogen is 0.1 EU or less;

(3) a process for producing a recombinant human serum albumin which comprises the steps of allowing the recombinant human albumin to contact with a carrier for hydrophobic chromatography at a pH of 2 to 5 and a salt concentration of 0.4 to 1 M and exposing the carrier to a pH of 6 to 8 and a salt concentration of 0.01 to 0.3 M;

(4) a process for producing a recombinant human serum albumin which comprises the steps of treating the recombinant human serum albumin with boric acid or a salt thereof to remove contaminants; and

(5) the process described in the above (4) which further comprises after the removal of contaminants treating the albumin with a ultrafiltration membrane having a molecular weight exclusive limit of about 100,000.

DETAILED DESCRIPTION OF THE INVENTION

(1) Recombinant HSA

The origin of the starting recombinant HSA used in the instant invention is not limited so long as the HSA is prepared by means of gene manipulation techniques. The HSA-producing host to be used in the instant invention is not limited so long as it has been prepared via gene manipulation techniques, hence the host can be selected from hosts already known in the art, as well as those hosts which will be developed in the future. Illustrative examples of the host include microbial cells, such as *Escherichia coli*, various yeast species, *Bacillus subtilis* and the like, and animal cells. Particularly preferred hosts are yeast species, especially those belonging to the genus *Saccharomyces*, such as *Saccharomyces cerevisiae*, or the genus *Pichia*, such as *Pichia pastoris*. Auxotrophic strains or antibiotic-sensitive strains also may be used. *Saccharomyces cerevisiae* AH22 (a, his 4, leu 2, can 1), *Pichia pastoris* GTS115 (his 4) and the like strains are used preferably. The HSA used in the instant invention is preferably produced using these hosts.

Preparation of the HSA-producing hosts, production of HSA by culturing the hosts and isolation and recovery of HSA from the resulting culture broth may be effected using known techniques or modified procedures thereof. For example, preparation of an HSA-producing host (or an HSA-producing strain) may be effected using a process in which a natural human serum albumin gene is used (JP-A-58-56684 corresponding to EP-A-73646, JP-A-58-90515 corresponding to EP-A-79739 and JP-A-58-150517 corresponding to EP-A-91527), a process in which a modified human serum albumin gene is used (JP-A-62-29985 and JP-A-1-98486 corresponding to EP-A-206733), a process in which a synthetic signal sequence is used (JP-A-1-240191 corresponding to EP-A-329127), a process in which a serum albumin signal sequence is used (JP-A-2-167095 corresponding to EP-A-319641), a process in which a recombinant plasmid is introduced into chromosome (JP-A-3-72889 corresponding to EP-A-399455), a process in which hosts are fused (JP-A-3-53877 corresponding to EP-A-409156), a process in which mutation is generated in a methanol containing medium, a process in which a mutant $AOX_2$ promoter is used (EP-A-506040), a process in which HSA is expressed in *B. subtilis* (JP-A-62-215393 corresponding to EP-A-229712), a process in which HSA is expressed in yeast (JP-A-60-41487 corresponding to EP-A-123544, JP-A-63-39576 corresponding to EP-A-248657 and JP-A-63-74493 corresponding to EP-A-251744) and a process in which HSA is expressed in *Pichia* (JP-A-2-104290 corresponding to EP-A-344459).

The process in which mutation is generated in a methanol-containing medium is carried out in the following manner.

Firstly, a plasmid containing a transcription unit which is constructed so as to express HSA under the control of AOX1 promoter is introduced into the AOX1 gene region of an appropriate host, preferably a *Pichia* yeast, more preferably *Pichia* strain GTS115 (NRRL deposition number Y-15851) (JP-A-2 104290 corresponding to EP-A-344459) to obtain a transformant. Since the thus obtained transformant does not grow well in a methanol-containing medium, mutation of the transformant is effected by culturing the transformant in a methanol-containing medium to isolate a mutant strain which is capable of growing in the medium. The methanol concentration in the medium may be in the range of approximately from 0.01 to 5%. The medium may be either synthetic or natural, and the culturing may be carried out at 15 to 40°C for 1 to 1,000 hours.

Culturing of an HSA-producing host (an HSA production process) may be carried out using known processes disclosed in the aforementioned references, or in accordance with a process disclosed in JP-A-3-83595 in which high concentration substrate inhibition of HSA producer cells is avoided by gradually adding a high concentration glucose solution to a medium by means of fed batch fermentation, thereby enabling production of both the producer cells and the product in high concentrations, or in accordance with another process disclosed in JP-A-4-293495 corresponding to EP-A-504823 in which productivity of HSA is improved by adding fatty acids to a medium.

Isolation and recovery of HSA may be carried out using known processes disclosed in the aforementioned references, or in accordance with a process disclosed in JP-A-3-103188 corresponding to EP-A-420007 in which proteases are inactivated by heat treatment or a coloration inhibition process disclosed in JP-A-4-54198 corresponding to U.S. Patent 5,132,404 or EP-A-464590 in which HSA is separated from coloring substances using at least one adsorbent selected from the group consisting of anion exchangers, hydrophobic carriers and activated charcoal.

The medium for culturing a transformed host may be prepared by adding fatty acids having 10 to 26 carbon atoms, or salts thereof, to a known medium, and culturing the transformant under known conditions.

The medium may be either synthetic or natural, but preferably a liquid medium. For example, a suitable synthetic medium may be composed of: carbon sources, such as various saccharides and the like; nitrogen sources, such as urea, ammonium salts, nitrates and the like; trace nutrients, such as various vitamins, nucleotides and the like; and inorganic salts, such as of Mg, Ca, Fe, Na, K, Mn, Co, Cu and the like. An illustrative example of such a medium is YNB liquid medium which consists of 0.7% Yeast Nitrogen Base (Difco) and 2% glucose. An illustrative example of a useful natural medium is YPD liquid medium which consists of 1% Yeast Extract (Difco), 2% Bacto Peptone (Difco) and 2% glucose. The medium pH may be neutral, weakly basic or weakly acidic. In the case of a methanol assimilating host, the medium may be further supplemented with methanol in an amount of approximately from 0.01 to 5%.

Culturing of a host may be carried out preferably at 15 to 43°C (20 to 30°C for yeast strains, 20 to 37°C for bacterial strains) for 1 to 1,000 hours, by means of static or shaking culturing or batch, semi-batch or continuous culturing under agitation and aeration.

In that instance, it is desirable to prepare a seed culture prior to the batch culturing. The seed culturing may be carried out using the aforementioned YNB liquid medium or YPD liquid medium, preferably at 30°C (yeast) or 37°C (bacterium) and for 10 to 100 hours.

After completion of, the culturing, HSA is recovered from the resulting culture medium or cells in the usual way.

### (2) Purification of HSA

The HSA to be subjected to the purification step according to the instant invention can be previously purified by the known method such as various fractionation, adsorption chromatography, gel filtration, density-gradient centrifugation or dialysis.

Suitable previous purification contains the following steps:

(i) treating a culture supernatant of a host which expresses human serum albumin, with a first ultrafiltration membrane having a molecular weight exclusive limit of from 100,000 to 500,000 and then with a second ultrafiltration membrane having a molecular weight exclusive limit of from 1,000 to 50,000 to yield a first filtrate;

(ii) heat-treating the first filtrate at 50 to 70°C for 30 minutes to 5 hours to yield a heated sample;

(iii) acid-treating the heated sample at a pH of from 3 to 5 to yield an acid-treated sample;

(iv) treating the acid-treated sample using an ultrafiltration membrane having a molecular weight exclusive limit of from 100,000 to 500,000 to yield a second filtrate;

(v) exposing the second filtrate to a cation exchanger at a pH of 3 to 5 and a salt concentration of 0.01 to 0.2 M, and then exposing said cation exchanger to a pH of 8 to 10 and a salt concentration of 0.2 to 0.5 M to yield a first eluate;

(vi) allowing the first eluate to contact with a carrier for hydrophobic chromatography at a pH of 6 to 8 and a salt concentration of 0.01 to 0.5 M, and recovering non-adsorbed fractions to yield a second eluate; and

(vii) allowing the second eluate to contact with an anion exchanger at a pH of 6 to 8 and a salt concentration of 0.01 to 0.1 M, and recovering non-adsorbed fractions to yield said albumin.

The above step (vi) may be replaced by the step in which the resulting eluate of the step (v) is allowed to contact with a carrier for hydrophobic chromatography at a pH of 6 to 8 and a salt concentration of 1 to 3 M and then the carrier is exposed to a pH of 6 to 8 and a salt concentration of 0.01 to 0.5 M.

The above step (vii) may be replaced by the step in which the resulting eluate of the step (vi) is allowed to contact with an anion exchanged at pH 6 to 8 and a salt concentration of 0.001 to 0.05 M, and then the anion exchanger is exposed to a pH 6 to 8 and a salt concentration of 0.05 to 1 M.

In addition, the above purification steps further comprises a salt precipitation step following step (v), step (vi) and step (vii) in which the salt precipitation step is carried out by exposing the first eluate, the second eluate or the albumin to a pH of 3 to 5 and a salt concentration of 0.5 to 3 M to yield a precipitate. The salt precipitation step may be effected after the below-described chelate resin treatment.

### (3) Decoloration of HSA (chelate resin treatment)

The above purification steps may further contain a step of decoloration of HSA, preferably as a final step, which is carried by allowing HSA to contact with a chelate resin which has a specified ligand moiety.

Preferably, the carrier moiety of the chelate resin may have hydrophobic nature. Examples of such a type of carrier moiety include a copolymer of styrene and divinylbenzene, a copolymer of acrylic acid and methacrylic acid end the like.

Examples of the ligand moiety include a thiourea group, as well as a polyamine group (including a polyalkylene polyamine group such as polyethylene polyamine or the like) which contains, in one molecule, a plurality of sub-groups consisting of a polyol group such as an N-methylglucamine group, an imino group, an amino group, an ethyleneimino group and the like. Illustrative examples of preferred commercially available chelate resins having the above-described carrier and ligand moieties, include DIAION CRB02® (ligand moiety, N-methylglucamine group, available from Mitsubishi Kasei Corp.), DIAION CR20® (ligand moiety, $-NH(CH_2CH_2NH)_nH$, available from Mitsubishi Kasei Corp.), LEWATIT TP214® (ligand moiety, $-NHCSNH_2$, available from Bayer) and AMBERLITE CG4000®, all of which have a copolymer of styrene and divinylbenzene as the carrier moiety.

Preferred conditions for the chelate resin treatment are as follows.

pH: acidic or neutral (pH 3 to 9, preferably 4 to 7),

period: at least 1 hour, preferably 6 hours or more,

ionic strength: 50 mmho or less, preferably 1 to 10 mmho,

mixing ratio: 0.1 to 100 g, preferably 1 to 10 g, of the resin based on 250 mg of HSA (wet basis).

(4) Hydrophobic chromatography

Free nonantigenic contaminants detectable by the phenol-sulfuric acid method are not fully removed from the HSA obtained through the above-described steps (i) to (vii) and the chelate resin treatment.

The HSA obtained through the above-described treatments is allowed to contact with a carrier for hydrophobic chromatography at a pH of 2 to 5, preferably 3 to 4 and a salt concentration of 0.4 to 1 M, preferably 0.4 to 0.7 M. The elution can be effected at a pH of 6 to 8, preferably 6.5 to 7 and a salt concentration of 0.01 to 0.3 M, preferably 0.05 to 0.2 M. The above-described step (vi) may be replaced with this hydrophobic chromatography step. Thus, HSA which does not contain free nonantigenic contaminants detectable by the phenol-sulfuric acid method can be recovered.

The term "phenol-sulfuric acid treatment" used herein means one of colorimetric determination of carbohydrates which comprises adding a phenol solution to a sample carbohydrate solution, adding concentrated sulfuric acid thereto, shaking the mixture to allow a furfural derivative derived from the carbohydrate utilizing heat of solution to react with phenol and colorimetrically determining the resulting colored reaction product. The free nonantigenic contaminants detectable by the phenol-sulfuric acid method include neutral carbohydrates such as pentose and hexose, monocarbohydrate glycoside such as oligosaccharides, complex carbohydrates and uronic acid, methylated carbohydrate and the like. These contaminants do not cause antigen-antibody reaction with antibodies against producer host-derived substances.

Carriers for use in hydrophobic chromatography include those containing an alkyl group (butyl group, octyl group, octyldecyl group and the like), each group having 4 to 18 carbon atoms, and those containing a phenyl group. Illustrative examples of the butyl group-containing carriers include butyl-agarose, butyl-polyvinyl (trade name, Butyl Toyopearl®, available from Tosoh Corp.) and the like, those of the octyl group-containing and octyldecyl group-containing carriers include octyl-agarose and octyldecyl-agarose, respectively, and those of the phenyl group-containing carrier include phenyl-cellulose (trade name, Phenyl Cellulofine®, available from Seikagaku Corp.) and the like.

HSA which does not contain free nonantigenic contaminants detectable by the phenol-sulfuric acid method can be obtained by a treatment with a ConA-immobilized carrier such as ConA-Sepharose (Pharmacia) and the like in place of this hydrophobic chromatography treatment. The ConA treatment can be carried out by contacting the HSA fraction with a ConA-immobilized carrier either in a batchwise or column method at a pH of 6 to 8 and a salt concentration of 0.01 to 0.1 M and recovering non-adsorbed fractions.

(5) Treatment with boric acid or a salt thereof

Instead of the above treatment (4), the HSA obtained through the above-described steps (i) to (vii) and the chelate resin treatment can be treated with boric acid or a salt thereof to remove antigenic producer host-derived contaminants and pyrogen as well as free nonantigenic contaminants detectable by the phenol-sulfuric acid method.

Examples of the boric acid include orthoboric acid, metaboric acid, tetraboric acid and the like. The salts thereof include alkali metal salts such as sodium salt and potassium salt, alkaline earth metal salts such as calcium salt, and the like. Calcium tetraborate is preferably used. Boric acid or a salt thereof is added to a final concentration of about 0.01 to 1 M, preferably about 0.05 to 0.2 M. This treatment can be carried out at a pH of about 8 to 11, preferably about 9 to 10 for about 1 to 10 hours. This treatment is preferably affected at a low electric conductivity, for example, 1 mS or less. The HSA concentration is preferably low, for example, 5% or less, more preferably about 0.1 to 3%.

After the treatment with boric acid or a salt thereof, the precipitate formed are removed by, for example, centrifugation or filtration and the supernatant is recovered, concentrated and desalted.

(6) Ultrafiltration

The HSA obtained after the above step (5) is preferably subjected to ultrafiltration using an ultrafiltration membrane having a molecular weight exclusive limit of about 100,000.

(7) Properties of purified recombinant HSA

The HSA of the instant invention is a homogeneous substance having a molecular weight of about 67,000 and an isoelectric point of 4.6 to 5.0. The HSA consists of a monomer and contains substantially no dimers, polymers or decomposed products. In fact, the total content of dimers, polymers and hydrolyzed products is approximately 0.01% or less. Also, the HSA of the instant invention contains substantially no producer host-derived contaminants, such as protein, polysaccharide and the like, which means contaminants having antigenicity. In the case of a 25 w/v% HSA solution, the content of the contaminants may be 1 ng/ml or below, preferably 0.1 ng/ml or below, and the polysaccharide content may be 1 ng/ml or below, preferably 0.1 ng/ml below. In that case, the purity of the HSA is calculated to be 99.999999% or more, preferably 99.9999999% or more. The degree of coloring of the 25 w/v% HSA solution may be in the range of from 0.01 to 0.05 in terms of an $A_{350}/A_{280}$ ratio, from 0.001 to 0.02 as an $A_{450}/A_{280}$ ratio and from 0.001 to 0.005 as an $A_{500}/A_{280}$ ratio. In addition, the amount of fatty acids linked to the HSA may be one molecule or less, preferably 0.1 molecule or less, per one HSA molecule.

The recombinant HSA according to the instant invention contain free nonantigenic contaminant detectable by the phenol-sulfuric acid method in an amount of only 1 $\mu$g or less per 250 mg of HSA. The nonantigenic contaminants means those which do not cause antigen-antibody reaction with antibodies against producer host-related substances. The recombinant HSA preferably further contains antigenic producer host-related contaminants in an amount of 0.1 ng or less and pyrogen in an amount of 0.1 EU or less.

An HSA preparation which is so safe that side effects attributed to the contaminants could be obviated can be provided using the HSA of the instant invention. Further, according to the method of the present invention, certain contaminants contained in or secreted by the host microorganism during cultivation, specifically, free nonantigenic contaminants detectable by the phenol-sulfuric acid method, antigenic producer host-derived contaminants and pyrogen can be fully removed from the recombinant HSA-containing fraction. Thus, the recombinant HSA obtained by the method of the present Invention is of very high purity.

The following examples are provided to further illustrate the instant invention. It is to be understood, however, that the examples are not to limit the scope of the instant invention.

REFERENCE EXAMPLE 1

(1) Used strain, *Pichia pastoris* GCP101

A strain of *Pichia pastoris*, PC4130, obtained in accordance with the process disclosed in JP-A-2-104290, was made by digesting a plasmid pPGP1, containing a transcription unit which is constructed so as to express HSA under the control of an AOX1 promoter, with *Nco*I and then substituting the resulting *Not*I-digested fragment for the AOX1 gene region of a *Pichia pastoris* strain GTS115 (his4). The strain does not grow well in a medium containing methanol as the carbon source (Mut⁻ strain) because of the deletion of the AOX1 gene.

The strain PC4130 was inoculated into 3 ml of YPD medium (1% yeast extract, 2% Bacto Peptone and 2% glucose). After 24 hours of culturing, the cells were inoculated into 50 ml of YPD medium so that the cell density should be adjusted to initial turbidity with an $OD_{540}$ of 0.1. After 3 days of culturing at 30°C, the resulting cells again were inoculated into 50 ml of YPD medium at an initial cell turbidity of 0.1 at $OD_{540}$. Thereafter, subculturing was repeated every 3 days in the same manner. After each subculturing, cells were diluted with sterile water and poured onto a 2% MeOH-YNBw/oa.a. plate (0.7% Yeast Nitrogen Base without Amino Acids, 2% methanol and 1.5% agar powder) in an inoculum size of $10^7$ cells/plate, followed by 5 days of culturing at 30°C to judge the present/absence of colonies. Twenty colonies were found on the 2% MeOH-YNBw/oa.a. plate after 12 days of the successive subculturing. Mut⁻ strains can hardly grow on the 2% MeOH-YNBw/oa.a. medium while Mut⁺ strains can grow well. That is, advent of a colony means that the strain acquired the capacity of increased methanol assimilation and thus a Mut⁺ strain was obtained. One of the thus obtained colonies was diluted appropriately with sterile water and spread onto a 2% MeOH-YNBw/oa.a. plate to isolate single colonies. One of the resulting single colonies was named GCP101.

(2) Culturing of the strain

(First seed culture)

A 1 ml portion of the strain which had been frozen in glycerol was inoculated into a 1,000 ml baffled Erlenmeyer flask containing 200 ml of YPD medium (see Table 1) and cultured at 30°C for 24 hours with shaking.

Table 1

| Composition of YPD medium | |
|---|---|
| Components | Concentration (g/L) |
| Yeast extract | 10 |
| Peptone | 20 |
| Glucose | 20 |

(Second seed culture)

The first seed culture broth was inoculated into a 10 liter-jar fermentor containing 5 liters of YPD medium, and the second seed culturing was carried out at 30°C for 24 hours with agitation and at an aeration rate of 5 liters per minutes. In the seed culturing, the pH of the medium was not controlled.

(Main culture)

The second seed culture broth was transferred into a 1,200 liter-fermentor containing 250 liters of a batch culture medium (see Table 2), and batch culturing was started with agitation and aeration under an internal pressure of 0.5 kg/cm² and at a maximum aeration rate of 800 liter/min under atmospheric pressure. The agitation rate was controlled so that the level of dissolved oxygen in the medium was maintained at approximately 50 to 30% of the saturated dissolved oxygen concentration. When the glycerol in the batch culture medium was consumed, addition of a feeding medium (see Table 3) was started. Feeding rate of the medium was controlled using a computer in such a manner that methanol did not accumulate in the culture medium, thereby effecting a high density culturing. The medium pH was controlled at a fixed level of 5.85 by the addition of 28% aqueous ammonia. For defoamation of the culture medium, an antifoam agent (Adecanol®, manufactured by Asahi Denka Kogyo K.K.) was added in an amount of 0.30 ml/liter at the time of the commencement of the batch culture, thereafter adding a small amount when required.

Table 2  Composition of batch culture medium

| Components | Amount per liter |
|---|---|
| Glycerol | 50.0 g |
| $H_3PO_4$ (85%) | 14.0 ml |
| $CaSO_4 \cdot 2H_2O$ | 0.6 g |
| $K_2SO_4$ | 9.5 g |
| $MgSO_4 \cdot 7H_2O$ | 7.8 g |
| KOH | 2.6 g |
| Biotin solution *1 | 1.6 ml |
| YTM solution *2 | 4.4 ml |

*1 Biotin solution: 0.2 g/l
*2 YTM solution:

| Components | Amount per liter |
|---|---|
| $FeSO_4 \cdot 7H_2O$ | 65.0 g |
| $CuSO_4 \cdot 5H_2O$ | 6.0 g |
| $ZnSO_4 \cdot 7H_2O$ | 20.0 g |
| $MnSO_4 \cdot 4-5H_2O$ | 3.0 g |
| $H_2SO_4$ | 5.0 ml |

Table 3

| Composition of feeding medium | |
|---|---|
| Components | Amount |
| YTM solution | 2 ml |
| Methanol | 1,000 ml |

REFERENCE EXAMPLE 2

An HSA expression plasmid pMM042 was constructed using an AOX2 promoter (a mutant of the natural AOX2 promoter (*YEAST*, 5, 167-177, 1988; *Mol. Cell. Biol.*, 9, 1316-1323, 1989), in which the 255th base upstream from the initiation codon of said promoter is changed from T to C) isolated from the strain GCP101 obtained in Reference Example 1. The thus constructed plasmid was introduced into *Pichia pastoris* GTS115 to obtain a transformant UHG42-3 (EF-A-506040). Thereafter, the thus obtained transformant was cultured in accordance with the procedure of Reference Example 1, thereby allowing the transformant to produce HSA.

REFERENCE EXAMPLE 3

[i] Isolation of culture supernatant - membrane fractions (I) and (II) -

About an 800 liter portion of the culture broth obtained in Reference Example 1 was subjected to a filter press to isolate the culture supernatant. The resulting supernatant subsequently was treated with an ultrafiltration membrane having a molecular weight exclusive limit of 300,000. Then, the resulting filtrate wan concentrated to a volume of about 80 liters using an ultrafiltration membrane having a molecular weight exclusive limit of 30,000 [membrane fraction (I)].

Next, the membrane fraction (I) was heat-treated at 60°C for 3 hours in the presence of 5 mM of sodium caprylate, 10 mM of cysteine and 100 mM of aminoguanidine at pH 7.5. The thus heat-treated solution was cooled dawn rapidly to about 15°C, adjusted to pH 4.5 and than treated with an ultrafiltration membrane having a molecular weight exclusive limit of 300,000 [membrane fraction (II)]. Thereafter, using an ultrafiltration membrane having a molecular weight exclusive limit of 30,000, the buffer in the resulting albumin solution was replaced by a 50 mM acetate buffer (pH 4.5) containing 50 mM of sodium chloride.

[ii] Cation exchanger treatment

The albumin solution obtained in the above step [i] was applied to a column packed with S-Sepharose® which had been equilibrated in advance with a 50 mM acetate buffer (pH 4.5) containing 50 mM of sodium chloride, the column was washed thoroughly with the same buffer and then elution was carried out with a 0.1 M phosphate buffer (pH 9) containing 0.3 M sodium chloride.

Polysaccharide content before and after the cation exchanger treatment was measured in accordance with the phenol-sulfuric acid method to find that the polysaccharide content has been reduced by 1/20 by this treatment.

[iii] Hydrophobic chromatography

The albumin solution eluted from the S-Sepharose® column was applied to a column packed with Phenyl Cellulofine® which has been equilibrated in advance with a 50 mM phosphate buffer (pH 6.8) containing 0.15 M sodium chloride. Since albumin does not adsorb to Phenyl Cellulofine® under such conditions, the albumin fractions which passed through the column were collected.

The albumin solution thus recovered was concentrated to a volume of about 50 liters using an ultrafiltration membrane having a molecular weight exclusive limit of 30,000, and at the same time, the buffer in the albumin solution was replaced by a 50 mM phosphate buffer (pH 6.8).

[iv] Anion exchanger treatment

The albumin solution thus treated with hydrophobic chromatography, concentrated and buffer-exchanged in the above step [iii] was applied to a column packed with DEAE-Sepharose® which had been equilibrated in advance with a 50 mM phosphate buffer (pH 6.8). Under such conditions, albumin was not adsorbed to the DEAE-Sepharose® but passed through the column.

[v] Salting-out of HSA

To a 5 w/v% HSA solution was added sodium chloride to a final concentration of 1 M. The resulting solution was adjusted to pH 3.5 with acetic acid to precipitate HSA, and the thus precipitated HSA was separated from the supernatant fluid by centrifugation, thereby effecting removal of impurities.

[vi] Decoloration

A 1 ml portion of the 25 w/v% solution of purified HSA wad mixed with 1 g of DIAION CRB02® (a chelate resin having a styrene-divinylbenzene copolymer as the carrier portion and an N-methylglucamine group as the ligand portion, manufactured by Mitsubishi Kasei Corp.), and the resulting mixture was stirred for 24 hours at room temperature at pH 6.8 and ionic strength of 5 mmho. The resin then was washed with distilled water to recover the non-absorbed HSA-containing fraction.

EXAMPLE 1

Sodium chloride was added to the yeast-derived HSA-containing solution obtained in Reference Example 1 (or Reference Example 2) and Reference Example 3 (without effecting salting-out [v]) to a final concentration of 0.5 M. The resulting solution was adjusted to a pH of 3.5 and applied to a column packed with Phenyl-Cellulofine. The column was washed with a 0.5 M sodium chloride solution (pH 3.5) and elution was carried out using 50 mM phosphate buffer (pH 6.8) containing 0.15 M sodium chloride.

EXAMPLE 2

The HSA concentration of the yeast-derived HSA-containing solution obtained in Reference Example 1 (or Reference Example 2) and Reference Example 3 (excluding the salting-out step [v]) was adjusted to 2.5 w/v% so that the electric conductivity became 1 mS or below. Calcium tetraborate was added to the resulting solution to a final concentration of 100 mM and a pH value of the solution was adjusted to 9.5. After allowing the solution to stand for 10 hours, the precipitate formed was removed to recover the supernatant which was then concentrated and desalted.

EXAMPLE 3

The HSA-containing solution obtained in Example 2 was treated with a ultrafiltration membrane having a molecular weight exclusive limit of about 100,000.

EXAMPLE 4

The HSA solution was treated in the same manner as in Example 2 except for adding sodium tetraborate to a final concentration of 100 mM in place of calcium tetraborate and adding calcium chloride to a final concentration of 100 mM.

TEST EXAMPLE 1

HSA fractions (run Nos. 1 to 6 shown in Table 4) obtained through the steps among the steps described in Reference Example 3 shown in Table 4 were examined for the following items. The treatment with a ConA-immobilized carrier was carried out by applying the HSA fraction to a ConA-Sepharose (Pharmacia) column which had been equilibrated with a 50 mM phosphate buffer (pH 6.8) and recovering non-adsorbed fractions.

(1) Determination of the content of free contaminants by the phenol-sulfuric acid method

The content of free contaminants in each HSA fraction was determined by the phenol-sulfuric acid method in the conventional manner. Thus, each HSA fraction was directly examined by the phenol-sulfuric acid method to determine the total content of the contaminants (the sum of the free contaminant content and the nonfree contaminant content). Separately, each HSA fraction was treated with ConA-Sepharose (Pharmacia) in the same manner as described above and non-adsorbed fractions containing HSA were subjected to the determination by the phenol-sulfuric acid method to determine the content of nonfree contaminants. A difference obtained by taking the latter from the former means the content of free contaminants. A standard curve was prepared using mannnan as a standard material. The results are shown in Table 4.

Table 4

| Run No. | Purification step | | | | | Free contaminant content ($\mu$g/250 mg of H-SA) |
|---|---|---|---|---|---|---|
| | [i]-[iv] | [v] | [vi] | Example | 1 ConA | |
| 1 | do | - | - | - | - | 1000 |
| 2 | do | do | - | - | - | 500 |
| 3 | do | - | do | - | - | 200 |
| 4 | do | do | do | - | - | <1 |
| 5 | do | do | - | do | - | <1 |
| 6 | do | - | - | - | do | <1 |

Note:

[i]-[iv] means the same step from isolation of culture supernatant to anion exchanger treatment as described in Reference Example 3.

[v] means the same step of chelate resin treatment as described as the step [vi] in Reference Example 3.

[vi] means the same step of salting-out as described as the step [v] in Reference Example 3.

Example 1 means hydrophobic chromatography.

(2) Confirmation of nonantigenicity

A culture supernatant of a yeast strain which does not produce HSA was partially purified in accordance with the purification process of the instant invention and the resulting purified HSA solution was used to immunize rabbits. Using an antiserum preparation obtained from the immunized rabbits, detection of yeast-derived components in the purified HSA solution was carried out by means of enzyme immunoassay (EIA). The sample was subjected to the measurement after adjusting the HSA concentration of 25% (250 mg/ml). As a result, no yeast-derived component higher than the detectable limit of 0.1 ng/ml was detected in the HSA fraction before and after the hydrophobic chromatography according to the present invention.

TEST EXAMPLE 2

The yeast-derived HSA solution (sample 1) obtained in Reference Examples 2 and 3 (excluding the salting-out step [v]) was adjusted to the HSA concentration of 25 w/v% so that electric conductivity of the solution became 1 mS or less. Calcium tetraborate was added thereto to a final concentration of 100 mM and a pH value was adjusted to 9.5. After allowing the mixture to stand for about 10 hours, the precipitate formed was removed to recover the supernatant which was concentrated and desalted (sample 2). The resulting HSA solution was treated with a ultrafiltration membrane having a molecular weight exclusive limit of 100,000 (sample 3). Samples 1, 2 and 3 were examined for the following items and the results are shown in Table 5.

(1) Recovery of HSA

The HSA content was determined by measuring the absorbance at 280 nm or by performing SDS-PAGE. As a result, recoveries of HSA in samples 2 and 3 were both 95% or more.

(2) Determination of contaminants by EIA

The yeast-derived components were detected in the same manner as in Test Example 1 (2). As a result, the content of the contaminants in sample 1 was 10 ng/ml and no contaminant higher that the detectable limit of 0.1 ng/ml was detected in samples 2 and 3.

11

(3) Determination of free contaminants by the phenol-sulfuric acid method

The content of free contaminants was determined in the same manner as in Example 1 (1).

(4) Determination of pyrogen

The content of pyrogen was determined using Endospecy (Seikagaku Corporation) in accordance with the manufacture's instruction attached to the product.

Table 5

| Sample No. | Purification step | HSA recovery | Contaminant content (per 250 mg of HSA) | | Pyrogen (per 250 mg of HSA) |
|---|---|---|---|---|---|
| | | | EIA | Phenol-sulfuric acid method (free) | |
| 1 | After decoloration | - | 10 ng | 700 $\mu$g | 2.9 EU |
| 2 | After boric acid salt treatment | ≧95% | <0.1 ng | <1 $\mu$g | <0.1 EU |
| 3 | After ultrafiltration | ≧95% | <0.1 ng | <1 $\mu$g | <0.1 EU |

**Claims**

1. A recombinant human serum albumin, wherein a content of free nonantigenic contaminants detectable by the phenol-sulfuric acid method is 1 $\mu$g or less per 250 mg of said albumin.

2. The recombinant human serum albumin according to claim 1, wherein a content of antigenic producer host-derived contaminants is 0.1 ng or less and a content of pyrogen is 0.1 EU or less.

3. A process for producing human serum albumin which comprises allowing recombinant human serum albumin to contact with a carrier for hydrophobic chromatography at a pH of 2 to 5 and a salt concentration of 0.4 to 1 M and exposing the carrier to a pH of 6 to 8 and a salt concentration of 0.01 to 0.3 M for eluting the albumin.

4. A process for producing human serum albumin which comprises treating recombinant human serum albumin with boric acid or a salt thereof and recovering the human serum albumin fraction which substantially contain no contaminant.

5. The process for producing human serum albumin according to claim 4 further comprising treating the human serum albumin fraction with a ultrafiltration membrane having a molecular weight exclusive limit of 100,000.

6. A process for producing human serum albumin which comprises contacting recombinant human serum albumin with ConA at a pH of 6 to 8 and a salt concentration of 0.01 to 0.1 M and recovering non-adsorbed fractions.

7. A process for producing a recombinant human serum albumin comprising the steps of:
   (1) treating a culture supernatant of a host which expresses human serum albumin, with a first ultrafiltration membrane having a molecular weight exclusive limit of from 100,000 to 500,000 and then with a second ultrafiltration membrane having a molecular weight exclusive limit of from 1,000 to 50,000 to yield a first filtrate;
   (2) heat-treating the first filtrate at 50 to 70 °C for 30 minutes to 5 hours to yield a heated sample;
   (3) acid-treating the heated sample at a pH of 3 to 5 to yield an acid-treated sample;
   (4) treating the acid-treated sample using an ultrafiltration membrane having a molecular weight exclusive limit of from 100,000 to 500,000 to yield a second filtrate;

(5) exposing the second filtrate to a cation exchanger at a pH of 3 to 5 and a salt concentration of 0.01 to 0.2 M, and then exposing said cation exchanger to a pH of 8 to 10 and a salt concentration of 0.2 to 0.5 M to yield a first eluate;

(6) allowing the first eluate to contact with a carrier for hydrophobic chromatography at a pH of 6 to 8 and a salt concentration of 0.01 to 0.5 M for yielding a second eluate;

(7) allowing the second eluate to contact with an anion exchanges at a pH of 6 to 8 and a salt concentration of 0.01 to 0.1 M, and recovering non-adsorbed fractions to yield said albumin; and

(8) contacting said albumin with a carrier for hydrophobic chromatography at a pH of 2 to 5 and a salt concentration of 0.4 to 1 M and exposing the carrier to a pH of 6 to 8 and a salt concentration of 0.01 to 0.3 M for eluting albumin.

8. A process for producing a recombinant human serum albumin comprising the steps of:

(1) treating a culture supernatant of a host which expresses human serum albumin, with a first ultrafiltration membrane having a molecular weight exclusive limit of from 100,000 to 500,000 and then with a second ultrafiltration membrane having a molecular weight exclusive limit of from 1,000 to 50,000 to yield a first filtrate;

(2) heat-treating the first filtrate at 50 to 70°C for 30 minutes to 5 hours to yield a heated sample;

(3) acid-treating the heated sample at a pH of 3 to 5 to yield an acid-treated sample;

(4) treating the acid-treated sample using an ultrafiltration membrane having a molecular weight exclusive limit of from 100,000 to 500,000 to yield a second filtrate;

(5) exposing the second filtrate to a cation exchanger at a pH of 3 to 5 and a salt concentration of 0.01 to 0.2 M, and then exposing said cation exchanger to a pH of 8 to 10 and a salt concentration of 0.2 to 0.5 M to yield a first eluate;

(6) allowing the first eluate to contact with a carrier for hydrophobic chromatography at a pH of 6 to 8 and a salt concentration of 0.01 to 0.5 M for yielding a second eluate;

(7) allowing the second eluate to contact with an anion exchanger at a pH of 6 to 8 and a salt concentration of 0.01 to 0.1 M, and recovering non-adsorbed fractions to yield said albumin; and

(8) treating said albumin with boric acid or a salt thereof to provide an albumin fraction.

9. A process for producing a recombinant human serum albumin comprising the steps of:

(1) treating a culture supernatant of a host which expresses human serum albumin, with a first ultrafiltration membrane having a molecular weight exclusive limit of from 100,000 to 500,000 and then with a second ultrafiltration membrane having a molecular weight exclusive limit of from 1,000 to 50,000 to yield a first filtrate;

(2) heat-treating the first filtrate at 50 to 70°C for 30 minutes to 5 hours to yield a heated sample;

(3) acid-treating the heated sample at a pH of 3 to 5 to yield an acid-treated sample;

(4) treating the acid-treated sample using an ultrafiltration membrane having a molecular weight exclusive limit of from 100,000 to 500,000 to yield a second filtrate;

(5) exposing the second filtrate to a cation exchanger at a pH of 3 to 5 and a salt concentration of 0.01 to 0.2 M, and then exposing said cation exchanger to a pH of 8 to 10 and a salt concentration of 0.2 to 0.5 M to yield a first eluate;

(6) allowing the first eluate to contact with a carrier for hydrophobic chromatography at a pH of 6 to 8 and a salt concentration of 0.01 to 0.5 M for yielding a second eluate;

(7) allowing the second eluate to contact with an anion exchanger at a pH of 6 to 8 and a salt concentration of 0.01 to 0.1 M, and recovering non-adsorbed fractions to yield said albumin; and

(8) contacting said albumin with ConA at a pH of 6 to 8 and a salt concentration of 0.01 to 0.1 M, and recovering non-adsorbed fractions to yield said albumin.

10. A process for producing a recombinant human serum albumin comprising the steps of:

(1) treating a culture supernatant of a host which expresses human serum albumin, with a first ultrafiltration membrane having a molecular weight exclusive limit of from 100,000 to 500,000 and then with a second ultrafiltration membrane having a molecular weight exclusive limit of from 1,000 to 50,000 to yield a first filtrate;

(2) heat-treating the first filtrate at 50 to 70°C for 30 minutes to 5 hours to yield a heated sample;

(3) acid-treating the heated sample at a pH of 3 to 5 to yield an acid-treated sample;

(4) treating the acid-treated sample using an ultrafiltration membrane having a molecular weight exclusive limit of from 100,000 to 500,000 to yield a second filtrate;

(5) exposing the second filtrate to a cation exchanger at a pH of 3 to 5 and a salt concentration of 0.01 to 0.2 M, and then exposing said cation exchanger to a pH of 8 to 10 and a salt concentration of 0.2 to 0.5 M to yield a first eluate;

(6) allowing the first eluate to contact with a carrier for hydrophobic chromatography at a pH of 6 to 8 and a salt concentration of 0.01 to 0.5 M for yielding a second eluate;

(7) allowing the second eluate to contact with an anion exchanger at a pH of 6 to 8 and a salt concentration of 0.01 to 0.1 M, and recovering non-adsorbed fractions to yield said albumin; and

(8) exposing said albumin to a chelate resin and recovering non-adsorbed fractions to yield albumin;

(9) treating said albumin with boric acid or a salt thereof to provide an albumin fraction; and

(10) treating said albumin fraction with an ultrafiltration membrane having a molecular weight exclusive limit of 100,000.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| P,X | EP-A-0 570 916 (THE GREEN CROSS CORP.) 24 November 1993<br>* The application in its entirety *<br>--- | 1,2 | C07K3/28<br>C12N15/14 |
| X | EP-A-0 524 681 (GIST-BROCADES) 27 January 1993<br>* Example 2 * | 1,2 | |
| Y | * the whole document *<br>--- | 1-3 | |
| Y | EP-A-0 319 067 (GIST-BROCADES) 7 June 1989<br>* the whole document *<br>----- | 1-3 | |

TECHNICAL FIELDS
SEARCHED      (Int.Cl.5)

C07K

The present search report has been drawn up for all claims

| Place of search | Date of completion of the search | Examiner |
|---|---|---|
| MUNICH | 8 June 1994 | Germinario, C |

EPO FORM 1503 03.82 (P04C01)